(19) Europäisches Patentamt
European Patent Office
Office européen des brevets

(11) Veröffentlichungsnummer: **0 490 039 A2**

## (12) EUROPÄISCHE PATENTANMELDUNG

(21) Anmeldenummer: **91117172.6**

(22) Anmeldetag: **09.10.91**

(51) Int. Cl.5: **C07C 33/042**

(30) Priorität: **08.12.90 DE 4039238**

(43) Veröffentlichungstag der Anmeldung:
**17.06.92 Patentblatt 92/25**

(84) Benannte Vertragsstaaten:
**BE DE ES FR GB IT NL**

(71) Anmelder: **HÜLS AKTIENGESELLSCHAFT**
**Patentabteilung / PB 15 - Postfach 13 20**
**W-4370 Marl 1(DE)**

(72) Erfinder: **Heinrich, Friedrich, Dr.**
**Leunaer Strasse 8**
**W-4370 Marl(DE)**
Erfinder: **Sridhar, Srinivasan, Dr.**
**Lembecker Pfad 52**
**W-4370 Marl(DE)**

(54) **Verfahren zum Entwässern eines Gemisches aus Propargylalkohol und Wasser.**

(57)
2.1 Zum Entwässern von PAA wird meist eine Hilfsflüssigkeit als Schleppmittel bei der Destillation oder als Lösemittel bei der Extraktion eingesetzt. Die bekannten Hilfsflüssigkeiten verlangen einen erhöhten technischen Aufwand, sind schwer zugänglich oder verfahrenstechnisch bedenklich. Das neue Verfahren soll Hilfsflüssigkeiten bereitstellen, die leicht zugänglich und technisch leicht handhabbar sind, und mit denen der PAA praktisch wasserfrei und frei von Hilfsflüssigkeit gewonnen werden kann.
2.2 Beim erfindungsgemäßen Verfahren wird ein Ether - wie 3-Methyltetrahydropyran, Methyltertiärbutylether oder Diisopropylether - als Hilfsflüssigkeit eingesetzt; bevorzugt wird ein und derselbe Ether sowohl zum Extrahieren als auch als azeotropes Schleppmittel verwendet. Der PAA wird praktisch frei von Wasser und Hilfsflüssigkeit gewonnen. Das Wasser enthält nur geringe Mengen von PAA und Hilfsflüssigkeit; die Hilfsflüssigkeit enthält nur geringe Mengen von PAA und Wasser.
2.3 Das Verfahren wird eingesetzt zur Gewinnung von wasserfreiem PAA.

**EP 0 490 039 A2**

Die Erfindung betrifft die Gewinnung von wasserfreiem Propargylalkohol aus wäßrigen Gemischen.

Die Erfindung bezweckt, die bisher bekannten Verfahren wirtschaftlicher zu gestalten.

Es ist bekanntlich schwierig, wasserfreien Propargylalkohol (PAA; Siedepunkt 115 °C bei Normaldruck) aus dem unter Normaldruck bei 97 °C siedenden wäßrigen Azeotrop, das 43 Massen-% PAA enthält, zu gewinnen. Deshalb ist vorgeschlagen worden, eine Hilfsflüssigkeit einzusetzen.

Aus DE-AS 1 002 324 ist bekannt, Derivate des Tetrahydrofurans (THF), z. B. 2,5-Dimethyl-THF, oder Benzol als Hilfsflüssigkeit zu verwenden. Beim Einsatz von Benzol als Schleppmittel gehen beträchtliche Mengen an PAA in das ausgekreiste Wasser über, das erneut destilliert werden muß. Demgegenüber liefert 2,5-Dimethyl-THF als Entziehungsflüssigkeit günstigere Ergebnisse. Weiter wird vorgeschlagen, eine Mischung aus Benzol und Essigsäureethylester als Wasserentziehungsmittel zu verwenden. Ein azeotrop siedendes Gemisch aus PAA und Wasser wird mit dem Wasserentziehungsmittel vermischt und in einer Fraktioniersäule zum Sieden gebracht. Das überdestillierte Wasserazeotrop wird kondensiert, wobei es sich in eine organische und eine wäßrige Schicht trennt. Die organische Schicht wird in die Säule zurückgeführt, das von PAA praktisch freie Wasser wird ausgeschleust. Nach dem völligen Abschleppen des Wassers steigt die Siedetemperatur des Gemisches aus Wasserentziehungsmittel und PAA. Aus diesem Gemisch wird dann das Wasserentziehungsmittel fraktioniert abdestilliert, wobei der PAA als wasserfreies Sumpfprodukt, das frei von Wasserentziehungsmittel ist, zurückbleibt.

Aus DE-PS 851 050 ist bekannt, den zu entwässernden Stoff mittels einer bestimmten Menge eines Lösemittels aus der wäßrigen Lösung zu extrahieren; das Lösemittel soll bei gewöhnlicher Temperatur mit Wasser nicht mischbar sein, aber mit Wasser ein azeotrop siedendes Gemisch bilden.

Der Extrakt wird destilliert, der zu gewinnende Stoff verbleibt in wasserfreier Form im Rückstand. Als Extraktionsmittel werden Substitutionsprodukte des THF genannt.

Von den vorgeschlagenen Hilfsflüssigkeiten sind die THF-Derivate schwer zugänglich und damit teuer. Benzol hat einen unbefriedigenden Wirkungsgrad und verlangt - wie auch Diethylether - einen erhöhten technischen Aufwand. Bei der Mischung aus Benzol und Ethylacetat ist es weiter bedenklich, den Ester in Gegenwart von Wasser über längere Zeit zu destillieren und im Kreislauf zu führen; durch Verseifen können Ethanol und Essigsäure entstehen. Bei Anwesenheit von Ethanol wird die Phasentrennung erschwert oder schließlich verhindert. Die Essigsäure gelangt in das Sumpfprodukt und muß vom PAA getrennt werden.

Damit stellt sich die Aufgabe, zum Entwässern eines Gemisches aus PAA und Wasser durch azeotrope Destillation unter Verwendung einer Hilfsflüssigkeit, die bei normaler Temperatur mit Wasser nicht mischbar ist, aber mit Wasser ein Azeotrop bildet, eine Hilfsflüssigkeit zu finden, die leicht zugänglich ist und unbedenklich eingesetzt werden kann.

Diese Aufgabe wird erfindungsgemäß gelöst durch Verwenden eines Ethers als Hilfsflüssigkeit und azeotropes Schleppmittel für das Wasser; bei der azeotropen Destillation wird das Wasser über Kopf abgeführt und der PAA als wasserfreies Sumpfprodukt gewonnen.

In vielen Fällen ist es zweckmäßig, den PAA aus dem PAA-Wasser-Gemisch zunächst mittels eines Ethers zu extrahieren, wobei die überwiegende Menge des Wassers im Raffinat verbleibt, und der Extrakt anschließend durch azeotrope Destillation entwässert wird.

Falls Extraktion und azeotrope Destillation gemeinsam durchgeführt werden, ist es zweckmäßig, ein und denselben Ether sowohl als Extraktionsmittel als auch als azeotropes Schleppmittel einzusetzen.

Die Extraktion und/oder die azeotrope Destillation können diskontinuierlich oder kontinuierlich durchgeführt werden. Als Ether wird bevorzugt 3-Methyltetrahydropyran (MTHP) oder Methyltertiärbutylether (MTBE) oder Diisopropylether (DIPE) eingesetzt.

Das erfindungsgemäße Verfahren hat folgende Vorteile:

- Die Wasserazeotrope sind praktisch frei von PAA.
- Die Wasserazeotrope bilden zwei Phasen, wobei die Wasserphase nur eine unbedeutende Menge an Schleppmittel enthält.
- Die Wasserazeotrope sieden deutlich niedriger als wasserfreier PAA, wodurch das Entwässern wirtschaftlich durchführbar wird; ein niedriges Rücklaufverhältnis und wenige Trennstufen sind hinreichend für die Destillation.
- Die Ether sind stabil, auch in Gegenwart von Wasser und bei ständiger Kreislauffahrweise.
- Die Extraktion und die Destillation lassen sich besonders wirtschaftlich durchführen; das Extraktionsmittel kann, ohne in reiner Form verdampft worden zu sein, direkt in die Extraktionsstufe zurückgeführt werden.
- Der PAA kann nicht nur wasserfrei, sondern auch frei von Hilfsflüssigkeit gewonnen werden.
- Das Schleppmittel ist leicht zugänglich und technisch leicht handhabbar.

Das erfindungsgemäße Verfahren wird anhand der Figuren weiter erläutert.

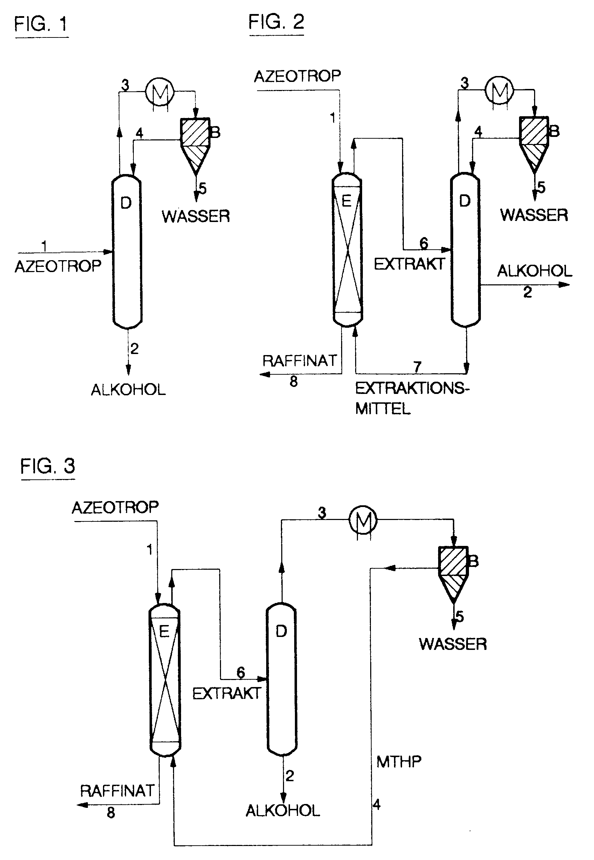

Die einfachste Ausführungsart für die azeotrope Destillation ist in Figur 1 dargestellt. Das PAA-Wasser-Azeotrop (Strom 1) wird in die Entwässerungskolonne (D) eingeleitet. Außerdem wird in diese Kolonne einmalig eine bestimmte Menge Ether gegeben. Der Dampf (Strom 3) wird kondensiert. Das Kondensat gelangt in den Trennbehälter (B), dort trennt es sich bei Normaltemperatur in zwei Phasen. Die (obere) organische Phase (Strom 4) wird zum Kopf der Kolonne (D) zurückgeführt, die (untere) Wasserphase (Strom 5) wird ausgeschleust. Dieser Strom wird gegebenenfalls etherfrei gestrippt; das dabei gewonnene Azeotrop aus Ether und Wasser wird in den Behälter (B) zurückgeführt. Der wasserfreie PAA erscheint im Sumpf der Kolonne (D). Bei kontinuierlicher und diskontinuierlicher Fahrweise geht diejenige Menge an Ether in den Ether-Kreislauf, die ausreicht, das Wasser abzuführen. Praktisch kein überschüssiger Ether gelangt in den Sumpf.

Bei dem Verfahren nach Figur 1 wird das gesamte Wasser durch azeotrope Destillation abgetrennt; dieses bedeutet die Verdampfung einer Wassermenge, die etwa der 1,3fachen Menge des PAA entspricht, sowie des entsprechenden azeotropen Anteils an Ether.

Das Verfahren nach Figur 1 kann deutlich verbessert werden, wenn der PAA zunächst durch Extrahieren weitgehend von Wasser befreit wird. Hierfür sind solche Hilfsflüssigkeiten besonders geeignet, die wenig Wasser aufnehmen und deutlich höher sieden als PAA, damit die thermische Trennung des PAA von der Hilfsflüssigkeit erleichtert wird und die Hilfsflüssigkeit selbst ohne Verdampfung in die Extraktionsstufe zurückgeführt werden kann.

Die Kombination aus Extraktion und Azeotrop-Destillation ist in Figur 2 dargestellt. In die Extraktionskolonne (E) werden das PAA-Wasser-Azeotrop (Strom 1) und die zur Extraktion benötigte Hilfsflüssigkeit (Strom 7) eingeleitet. Aus dem Sumpf der Kolonne wird das Raffinat (Strom 8) abgezogen. Am Kopf der Kolonne wird der flüssige Extrakt (Strom 6) abgezogen, der aus PAA, Extraktionsmittel und etwas Wasser besteht. Der Extraktstrom wird der Destillationskolonne (D) etwa in der Mitte zugeführt; in der Kolonne (D) wird der Extrakt in die drei Bestandteile zerlegt. Aus dem Kolonnensumpf wird die Hilfsflüssigkeit (Strom 7) abgezogen und als Extraktionsmittel in die Kolonne (E) zurückgeführt. Der PAA erscheint im Seitenstrom (Strom 2). Am Kopf der Kolonne (D) wird der Dampf (Strom 3) abgezogen, der kondensiert und in den Trennbehälter (B) geleitet wird; dort trennt sich das Kondensat bei Normaltemperatur in zwei Phasen. Die (obere) organische Phase (Strom 4) besteht fast nur aus der Hilfsflüssigkeit; dieser Strom wird auf den Kopf der Kolonne (D) als azeotropes Schleppmittel zurückgeleitet. Die (untere) Wasserphase (Strom 5) wird ausgeschleust. Bei diesem Verfahren erscheint der größte Teil des Wassers im Raffinat (Strom 8). Nur der geringe Anteil an Wasser, der in den Extrakt mitgeschleppt wird, ist in der Kolonne (D) durch azeotrope Destillation zu entfernen. Das Raffinat kann in die (in Figur 2 nicht dargestellte) Vorstufe zurückgeführt werden, wo es zum PAA-Wasser-Azeotrop aufdestilliert wird. Das PAA-Wasser-Azeotrop wird in den Strom (1) geleitet, das praktisch PAA-freie Wasser wird ausgeschleust.

Der PAA (Strom 2 in Figur 2) ist zwar wasserfrei, er kann jedoch geringe Mengen an Hilfsflüssigkeit enthalten. Von dieser Hilfsflüssigkeit, die höher siedet als der PAA, wird der PAA in einer weiteren Destillationstufe befreit.

Zur Gewinnung von PAA, der wasserfrei und frei von Hilfsflüssigkeit ist, ist das Verfahren gemäß Figur 3 geeignet. Dazu wird eine Hilfsflüssigkeit eingesetzt, deren Siedetemperatur unter der Siedetemperatur des PAA liegt.

Das PAA-Wasser-Azeotrop (Strom 1) wird auf den Kopf der Extraktionskolonne (E) gegeben, die Hilfsflüssigkeit (Strom 4) wird am Fuß der Kolonne (E) zugeführt. Aus der Kolonne werden das Raffinat (Strom 8) am Fuß und der Extrakt (Strom 6) am Kopf entnommen. Der Extrakt wird in die Destillationskolonne (D) geleitet, in der er durch azeotrope Destillation getrennt wird. Der Dampf (Strom 3) wird kondensiert und in den Trennbehälter (B) geleitet. Die organische Phase (Strom 4) wird in die Kolonne (E) geleitet, das Wasser (Strom 5) wird ausgeschleust. Der PAA (Strom 2) wird aus dem Sumpf der Kolonne (D) entnommen, nachdem er durch Destillation von der Hilfsflüssigkeit befreit worden ist.

Die Hilfsflüssigkeit wirkt in der Kolonne (E) als Extraktionsflüssigkeit, in der Kolonne (D) als azeotropes Schleppmittel.

Das erfindungsgemäße Verfahren wird anhand der folgenden Beispiele weiter erläutert.

Beispiel 1: Azeotrope Destillation mittels eines Ethers (MTHP) als Schleppmittel

540 g PAA und 660 g Wasser werden mit 153 g MTHP (Siedepunkt 108 °C bei Normaldruck) versetzt und in einem Rundkolben vorgelegt. Das Gemisch wird bei Normaldruck in einer auf den Kolben aufgesetzten Kolonne mit 40 mm Nennweite und 65 cm hoher Laborpackung Typ EX aus V4A (Sulzer) bei dem Rücklaufverhältnis 20 destilliert. Der im Destillat anfallende Ether (obere Phase) wird laufend wieder dem Sumpf im Kolben zugeführt. Der Destillationsverlauf ist in Tabelle 1a dargestellt. Die untere Wasserphase der relevanten Fraktionen 1 bis 8 ist frei von PAA und kann ohne Produktverlust ausgeschleust werden. Die geringe Menge an verlorenem Schleppmittel wird ersetzt. Das MTHP wird anschließend durch einfache Fraktionierung als dessen Azeotrop mit dem PAA von PAA vollständig (unterhalb der Nachweisgrenze) abgetrennt. Die Fraktionen sowie der Rückstand bestehen aus 99 Massen-% PAA. Die Analysenergebnisse sind in Tabelle 1b zusammengestellt.

Beispiel 2: Extraktion mittels Tributylphosphat als Extraktionsflüssigkeit

197 g eines Gemisches aus 43 Massen-% PAA und 57 Massen-% Wasser werden mit 193 g Tributylphosphat (TBP) bei Normaltemperatur vermischt und 1 Minute im Scheidetrichter geschüttelt. Nach 5 Minuten trennt sich das Gemisch in zwei Phasen. 120 g der unteren Wasserphase (Raffinat) werden abgetrennt und wieder mit 193 g TBP, wie oben beschrieben, extraktiv behandelt. 88 g des erneut angefallenen Raffinats werden wieder mit 194 g TBP extrahiert. Der gesamte Vorgang entspricht einer dreifachen Kreuzstromextraktion. Das endgültige Raffinat (66 g) enthält 99,3 Massen-% Wasser neben 0,7 Massen-% PAA. TBP kann nicht nachgewiesen werden. Die Extrakte werden vereint und wie im Beispiel 1 mittels MTHP entwässert.
Die Ergebnisse sind in Tabelle 2 zusammengestellt.

Beispiel 3: Extraktion mittels Ethylhexanol als Extraktionsflüssigkeit

196 g eines Gemisches aus 43 Massen-% PAA und 57 Massen-% werden mit 164 g 2-Ethylhexanol (EH; Siedepunkt 185 °C bei Normaldruck) bei Normaltemperatur und wie im Beispiel 2 extrahiert. Nach zwei weiteren Extraktionen enthält das endgültige Raffinat (97,5 g) 86,4 Massen-% Wasser neben 0,3 Massen-% EH und 13,3 Massen-% PAA. Die vereinten Extrakte werden wie im Beispiel 1 mittels MTHP entwässert.
Die Ergebnisse sind in Tabelle 3 zusammengestellt.

Beispiel 4: Extraktion und Destillation mittels eines Ethers (MTHP) als Extraktionsflüssigkeit und azeotropes Schleppmittel

196 g eines Gemisches aus 43 Massen-% PAA und 57 Massen-% Wasser werden mit 172 g MTHP bei Normaltemperatur vermischt und wie im Beispiel 2 extrahiert. Nach zwei weiteren Extraktionen enthält das endgültige Raffinat (84 g) 96,1 Massen-% Wasser neben 2,1 Massen-% PAA und 1,8 Massen-% MTHP. Die Extrakte werden vereint und wie in Beispiel 1 mittels MTHP, aber ohne weiteren Zusatz, entwässert.
Auch bei einer kontinuierlichen Gegenstromextraktion ist ein Zusatz von MTHP zur Entwässerung nicht erforderlich.
Die Ergebnisse sind in Tabelle 4 zusammengestellt.

Beispiel 5: Azeotrope Destillation mittels eines Ethers (MTBE) als Schleppmittel

493 g eines Gemisches aus 43 Massen-% PAA und 57 Massen-% Wasser werden mit 2495 g MTBE (Siedepunkt 55,3 °C bei Normaldruck) versetzt und gemäß Beispiel 1 entwässert. Das Destillat ist frei von PAA. 13 % des Wassers werden in diesem Schritt als Destillat abgetrennt. Der Rückstand besteht aus zwei Phasen. Die größere obere Phase (20 % vom Einsatz) wird nach Zusatz von 7600 g MTBE durch eine weitere Destillation in einer Kolonne mit der Nennweite 50 mm und 75 cm hoher Laborpackung Typ EX aus V4A (Sulzer) bei dem Rücklaufverhältnis 25 und Normaldruck von Wasser und Ether befreit, wobei ein wasser- und etherfreier PAA erhalten wird.
Die Ergebnisse sind in Tabelle 5 zusammengestellt.

Beispiel 6: Extraktion mittels eines Ethers (MTBE) als Extraktionsflüssigkeit

195 g eines Gemisches aus 43 Massen-% PAA und 57 Massen-% Wasser werden mit 147 g MTBE bei Normaltemperatur vermischt und wie in den Beispielen 2 und 4 extrahiert. Die Extrakte werden vereint und wie in Beispiel 1 entwässert.
Die Ergebnisse sind in Tabelle 6 zusammengestellt.

Beispiel 7: Azeotrope Destillation mittels eines Ethers (DIPE) als Schleppmittel

246 g eines Gemisches aus 43 Massen-% PAA und 57 Massen-% Wasser werden mit 3864 g DIPE (Siedepunkt 68,5 °C bei Normaldruck) versetzt und gemäß Beispiel 1 entwässert. Das Destillat ist frei von PAA. Nach dem Azeotrop wird der Ether in einer Kolonne mit der Nennweite 40 mm, gefüllt mit 1 m hohem Maschendraht bei Normaldruck abdestilliert. Als Rückstand verbleiben 100 g PAA der gleichen Reinheit wie in Beispiel 1. PAA wird in beiden Phasen nicht nachgewiesen.

Die Ergebnisse sind in Tabelle 7 zusammengestellt.

Tabelle 1a (zu Beispiel 1)

| Nr. der Fraktion | Temperatur °C | | Druck hPa | Abgenommene Wassermenge (bezogen auf Einsatz % | Destillat-abnahme (bezogen auf Einsatz % |
|---|---|---|---|---|---|
| | Sumpf | Kopf | | | |
| 1 | 91 | 81 | 1013 | 0,47 | 3,83 |
| 2 | 93 | 82 | | 6,32 | 6,30 |
| 3 | 95 | 82 | | 13,08 | 13,67 |
| 4 | 93 | 82 | | 19,19 | 19,74 |
| 5 | 94 | 82 | | 29,80 | 30,35 |
| 6 | 101 | 82 | | 41,25 | 44,14 |
| 7 | 109 | 82 | | 43,85 | 46,74 |
| 8 | 110 | 82 | | 44,42 | 48,17 |
| 9 | 57 | 41 | 100 | 44,67 | 52,06 |
| 10 | 58 | 54 | | - | 56,93 |
| 11 | 57 | 55 | | - | 58,42 |
| 12 | 57 | 55 | | - | 60,20 |
| 13 | 58 | 55 | | - | 61,43 |
| 14 | 57 | 55 | | - | 63,60 |
| 15 | 56 | 55 | | - | 64,82 |
| 16 | 56 | 55 | | - | 66,06 |
| 17 | 56 | 55 | | - | 67,36 |
| | | | | Rückstand 335,42 g | 90,48 |
| | | | | Falle 8,73 g | 91,05 |
| | | | | Kolonnenin-halt und Verluste 129,96 g | 100,00 |

Tabelle 1b (zu Beispiel 1)

| Nr. der Fraktion | GC-Analyse Massen-% | | | | | | | |
|---|---|---|---|---|---|---|---|---|
| | Wasserphase | | | | organische Phase | | | |
| | $H_2O$ | MTHP | PAA | unbek. Rest | $H_2O$ | MTHP | PAA | unbek. Rest |
| 1 | 98,2 | 1,23 | - | 0,57 | | | | |
| 2 | 97,9 | 1,83 | - | 0,27 | | | | |
| 3 | 98,2 | 1,62 | - | 0,18 | | | | |
| 4 | 98,4 | 1,42 | - | 0,18 | | | | |
| 5 | 98,7 | 1,21 | - | 0,09 | | | | |
| 6 | n.a. | n.a. | n.a. | | | | | |
| 7 | 98,4 | 1,56 | - | 0,04 | | | | |
| 8 | 98,3 | 1,53 | - | 0,17 | | | | |
| 9 | 98,9 | 0,75 | 0,35 | - | 2,3 | 93,9 | 0,53 | 3,27 |
| 10 | - | - | - | - | 42,7 | 0,74 | 56,2 | 0,36 |
| 11 | | | | | 4,8 | - | 94,7 | 0,50 |
| 12 | | | | | 0,77 | 0,25 | 98,6 | 0,38 |
| 13 | | | | | 0,25 | - | 99,4 | 0,35 |
| 14 | | | | | 0,21 | - | 99,5 | 0,29 |
| 15 | | | | | 0,32 | - | 99,5 | 0,18 |
| 16 | | | | | 0,23 | - | 99,6 | 0,17 |
| 17 | | | | | 0,25 | - | 99,6 | 0,15 |
| | | | | | -[*] | -[*] | 99,3 | 0,70 |

[*] $H_2O$ und Ether nicht nachweisbar (< 0,1 %)

n.a. = nicht analysiert

Tabelle 2 (zu Beispiel 2)

| Stufe | Mengen [g] | | | | | Analyse [Massen-%] | | | | |
|---|---|---|---|---|---|---|---|---|---|---|
| | Einwaage | | Auswaage | | | Extrakt | | | Raffinat nach 3. Stufe | |
| | Gemisch PAA/$H_2O$ bzw. Raffinat aus der vorherigen Stufe | TBP | untere Phase | obere Phase | Zwischen-phase | $H_2O$ | TBP | PAA | $H_2O$ | PAA |
| 1 | 196,67 | 192,77 | 120,69 | 267,60 | - | 5,8 | 73,06 | 21,14 | | |
| 2 | 119,76 | 193,37 | 88,20 | 223,60 | - | 5,3 | 86,19 | 8,51 | | |
| 3 | 88,05 | 194,34 | 66,07 | 209,22 | 7,18 | 6,2 | 91,72 | 2,08 | 99,3 | 0,7 |

EP 0 490 039 A2

Tabelle 3 (zu Beispiel 3)

| Stufe | Mengen [g] | | | | Analyse [Massen-%] | | | | | |
|---|---|---|---|---|---|---|---|---|---|---|
| | Einwaage | | Auswaage | | Extrakt | | | Raffinat nach 3. Stufe | | |
| | Gemisch PAA/$H_2O$ bzw. Raffinat aus der vorherigen Stufe | EH | untere Phase | obere Phase | $H_2O$ | EH | PAA | $H_2O$ | PAA | EH |
| 1 | 195,97 | 164,21 | 146,61 | 212,53 | 5,4 | 77,19 | 17,41 | | | |
| 2 | 146,50 | 164,48 | 115,96 | 193,58 | 4,0 | 84,96 | 11,04 | | | |
| 3 | 115,85 | 164,02 | 97,50 | 181,55 | 3,2 | 90,42 | 6,38 | 86,4 | 13,3 | 0,3 |

EP 0 490 039 A2

Tabelle 4 (zu Beispiel 4)

| Stufe | Mengen [g] | | | | | Analyse [Massen-%] | | | | | |
|---|---|---|---|---|---|---|---|---|---|---|---|
| | Einwaage | | Auswaage | | | Extrakt | | | Raffinat nach 3. Stufe | | |
| | Gemisch PAA/$H_2O$ bzw. Raffinat aus der vorherigen Stufe | MTHP | untere Phase | obere Phase | Zwischen-phase | $H_2O$ | MTHP | PAA | $H_2O$ | PAA | MTHP |
| 1 | 196,18 | 172,07 | 113,11 | 254,05 | 30,81 | 6,4 | 70,0 | 23,6 | | | |
| 2 | 113,01 | 171,71 | 91,40 | 192,40 | 32,23 | 2,6 | 88,5 | 8,92 | | | |
| 3 | 91,32 | 172,67 | 83,65 | 179,50 | 31,79 | 1,6 | 95,0 | 3,40 | 96,1 | 2,1 | 1,8 |

EP 0 490 039 A2

Tabelle 5 (zu Beispiel 5)

| Fraktion | Temperatur | | Destillationsanteil bezogen auf Einsatz | | Analyse [Massen-%] | | | | | | | |
| | | | obere Phase | untere Phase | obere Phase | | | | untere Phase | | | |
| | Sumpf °C | Kopf °C | Massen-% | Massen-% | $H_2O$ | PAA | MTBE | weit. Komp. | $H_2O$ | PAA | MTBE | $CH_3OH$ |
|---|---|---|---|---|---|---|---|---|---|---|---|---|
| 1 | 54 | 49,5 | | | 0,72 | < 0,01 | 99,26 | 0,02 | 92,2 | < 0,001 | 2,6 | 5,2 |
| 2 | 54 | 49,5 | 12,25 | 0,14 | | | | | | | | |
| 3 | 54 | 50 | | | - | < 0,01 | 99,80 | 0,20 | 94,8 | < 0,001 | 2,7 | 2,5 |
| 4 | 54 | 50 | 12,49 | 0,22 | | | | | | | | |
| 5 | 55 | 50 | | | - | < 0,01 | 99,94 | 0,05 | 91,2 | < 0,001 | 2,7 | 6,1 |
| 6 | 55 | 50 | 14,27 | 0,28 | | | | | | | | |
| 7 | 56 | 50 | | | - | < 0,01 | 99,98 | 0,02 | 97,2 | < 0,001 | 2,6 | 0,2 |
| 8 | 57 | 50 | 12,36 | 0,23 | | | | | | | | |
| 9 | 59 | 50 | 12,39 | 0,24 | - | < 0,01 | 100 | - | 97,6 | < 0,001 | 2,4 | 0,04 |
| 10 | 62,5 | 50 | 11,86 | 0,20 | - | < 0,01 | 100 | - | 97,3 | < 0,001 | 2,7 | - |
| Kühl-falle | | | 0,88 | - | | | | | | | | |
| Rück-stand | | | 20,19 | 2,0 | - | 50,9 | 48,4 | 0,7 | 58,1 | 31,0 | 10,5 | 0,43 |

EP 0 490 039 A2

Tabelle 6 (zu Beispiel 6)

| Stufe | Mengen [g] | | | | | Analyse [Massen-%] | | | | | |
| | Einwaage | | Auswaage | | | Extrakt | | | Raffinat nach 3. Stufe | | |
| | Gemisch PAA/$H_2O$ | MTBE | untere Phase | obere Phase | Zwischen-phase | $H_2O$ | MTBE | PAA | $H_2O$ | MTBE | PAA |
| 1 | 195,46 | 146,62 | 115,27 | 223,98 | - | 10,3 | 60,21 | 29,49 | | | |
| 2 | 114,18 | 148,43 | 91,86 | 168,28 | - | 3,5 | 85,75 | 10,75 | | | |
| 3 | 91,75 | 146,60 | 82,92 | 150,92 | 2,51 | 1,9 | 94,61 | 3,49 | 95,5 | 1,9 | 2,6 |

EP 0 490 039 A2

Tabelle 7 (zu Beispiel 7)

| Fraktion | Rücklaufverhältnis | Temperatur Sumpf °C | Temperatur Kopf °C | Destillationsanteil bezogen auf Einsatz Obere Phase Massen-% | Destillationsanteil bezogen auf Einsatz Untere Phase Massen-% | Analyse [Massen-%] Obere Phase C$_6$-KW | Analyse [Massen-%] Obere Phase H$_2$O | Analyse [Massen-%] Obere Phase DIPE | Analyse [Massen-%] Untere Phase H$_2$O | Analyse [Massen-%] Untere Phase DIPE |
|---|---|---|---|---|---|---|---|---|---|---|
| 1 | 10 | 70 | 62 | 8,68 | 0,31 | 3,6 | 0,27 | 96,1 | 98,4 | 1,32 |
| 2 | | 70 | 62 | 8,61 | 0,37 | 1,79 | 0,29 | 97,9 | 99,0 | 0,90 |
| 3 | | 70,5 | 62 | 8,80 | 0,38 | 1,27 | 0,29 | 98,4 | 98,7 | 1,25 |
| 4 | | 71 | 62 | 9,21 | 0,40 | 0,88 | 0,29 | 98,7 | 98,7 | 1,21 |
| 5 | | 71,5 | 62 | 9,36 | 0,41 | 0,57 | 0,29 | 99,1 | 98,7 | 1,30 |
| 6 | | 72 | 63 | 8,99 | 0,39 | 0,36 | 0,30 | 99,3 | 98,9 | 1,08 |
| 7 | 25 | 72,5 | 64 | 2,98 | 0,12 | 0,31 | 0,31 | 99,3 | 98,7 | 1,27 |
| 8 | | 72,5 | 65 | 1,64 | 0,10 | 0,27 | 0,30 | 99,4 | 98,5 | 1,47 |
| 9 | | 73 | 66,5 | 2,46 | 0,09 | 0,22 | 0,30 | 99,5 | 98,2 | 1,77 |
| 10 | | 73 | 67,5 | 2,22 | 0,06 | 0,17 | 0,30 | 99,4 | 98,4 | 1,50 |
| 11 | | 73 | 68 | 1,91 | 0,0002 | 0,13 | 0,29 | 99,6 | 98,9 | 0,95 |
| 12 | | 73,5 | 68,5 | 3,38 | - | 0,10 | 0,31 | 99,6 | - | - |

(Restliche Komponenten < 0,1 % sind nicht angegeben)

## Patentansprüche

1. Verfahren zum Entwässern eines Gemisches aus Propargylalkohol und Wasser durch azeotrope Destillation unter Verwendung einer Hilfsflüssigkeit, die bei normaler Temperatur mit Wasser nicht mischbar ist, aber mit Wasser ein Azeotrop bildet, dadurch gekennzeichnet, daß
   - ein Ether als Hilfsflüssigkeit und azeotropes Schleppmittel für das Wasser eingesetzt wird, das Wasser bei der azeotropen Destillation über Kopf abgeführt wird und der Propargylalkohol als wasserfreies Sumpfprodukt gewonnen wird.

2. Verfahren nach Anspruch 1, dadurch gekennzeichnet, daß
   - ein Ether als Hilfsflüssigkeit und Extraktionsmittel eingesetzt wird, mit dem der Propargylalkohol zunächst aus dem Propargylalkohol-Wasser-Gemisch extrahiert wird, wobei die überwiegende Menge des Wassers im Raffinat verbleibt, und der Extrakt anschließend durch azeotrope Destillation entwässert wird.

3. Verfahren nach den Ansprüchen 1 und 2, dadurch gekennzeichnet, daß
   - ein und derselbe Ether sowohl als Extraktionsmittel als auch als azeotropes Schleppmittel eingesetzt wird.

4. Verfahren nach den Ansprüchen 1 bis 3, dadurch gekennzeichnet, daß
   - die Extraktion und/oder die azeotrope Destillation diskontinuierlich oder kontinuierlich durchgeführt werden.

5. Verfahren nach den Ansprüchen 1 bis 4, dadurch gekennzeichnet, daß
   - 3-Methyltetrahydropyran oder
   - Methyltertiärbutylether oder
   - Diisopropylether
als Ether eingesetzt wird.

FIG. 1

FIG. 2

FIG. 3